# FASCICULE DE BREVET EUROPEEN

(11) **EP 0 663 905 B1**
(45) Date de publication et mention de la délivrance du brevet: **15.01.1997**
(21) Numéro de dépôt: 93921980.4
(22) Date de dépôt: 04.10.1993
(51) Int. Cl.: C07D 305/14, C07D 413/12, C07C 271/22, C07D 263/04

(54) **NOUVEAU PROCEDE D'ESTERIFICATION DE LA BACCATINE III ET DE LA DESACETYL-10 BACCATINE III**
NEUES VERFAHREN ZUR VERESTERUNG VON BACCATIN III UND 10-DEACETYLBACCATIN III
NOVEL METHOD OF ESTERIFICATION OF BACCATINE III AN 10-DEACETYLBACCATINE III

(30) Priorité: 05.10.1992 FR 9211739
(43) Date de publication de la demande: 26.07.1995
(73) Titulaire: RHONE-POULENC RORER S.A., 92165 Antony Cédex (FR)
(72) Inventeur: DENIS, Jean-Noêl, F-38410 Uriage (FR); GREENE, Andrew, F-38410 Uriage (FR); MAS, Jean-Manuel, F-69100 Villeurbanne (FR)
(74) Mandataire: Pilard, Jacques
(86) Numéro de dépôt international: FR9300965
(87) Numéro de publication internationale: WO9407876

(56) Documents cités:
- EP-A- 0 336 840
- WO-A-92/09589

## Description

La présente invention concerne la préparation d'esters de la baccatine III et de la désacétyl-10 baccatine III de formule générale : 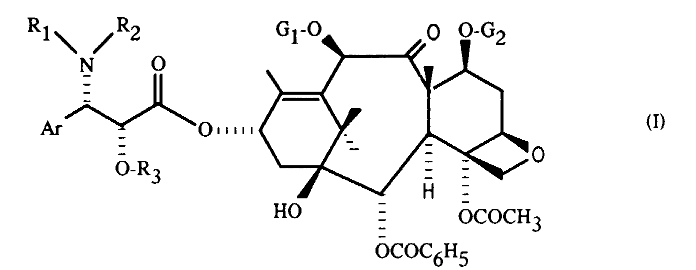 par estérification de la baccatine III ou de la désacétyl-10 baccatine III convenablement protégée de formule générale : 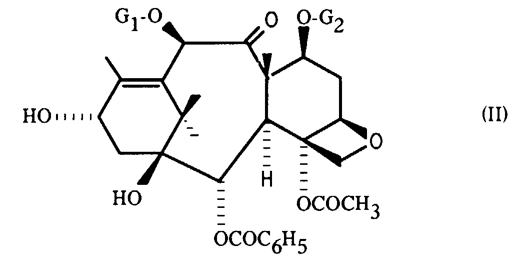 au moyen d'un acide activé de formule générale :

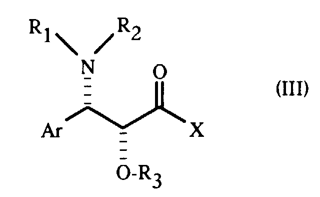

Dans les formules générales (I), (II) et (III), les différents symboles sont définis de la manière suivante :
- Ar représente un radical aryle éventuellement substitué,
- ou bien a) R₁ représente un radical aroyle éventuellement substitué ou un radical de formule R₄-O-CO-dans laquelle R₄ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclique azoté saturé ou non saturé contenant 4 à 6 chaînons et éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₂ représente un atome d'hydrogène, et
R₃ représente un groupement protecteur de la fonction hydroxy,
ou bien b) R₁ étant défini comme ci-dessus et pouvant en outre représenter un atome d'hydrogène, R₂ et R₃ forment ensemble un hétérocycle saturé à 5 ou 6 chaînons,
G₁ représente un radical acétyle ou un groupement protecteur de la fonction hydroxy,
G₂ représente un groupement protecteur de la fonction hydroxy, et
X représente un radical acyloxy ou aroyloxy ou un atome d'halogène.

Plus particulièrement, Ar et la portion aryle du radical aroyle représenté par R₁, identiques ou différents, représentent un radical phényle ou α- ou β-naphtyle éventuellement substitué, les substituants pouvant être choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, les radicaux alcényles et alcynyles contiennent 3 à 8 atomes de carbone et que les radicaux aryles sont des radicaux phényles ou α- ou β-naphtyles.

Plus particulièrement encore, Ar et la portion aryle du radical aroyle représenté par R₁, identiques ou différents, représentent un radical phényle éventuellement substitué par un atome de chlore ou de fluor ou par un radical alcoyle (méthyle), alcoyloxy (méthoxy), dialcoylamino (diméthylamino), acylamino (acétylamino) ou alcoyloxycarbonylamino (t.butoxycarbonylamino).

Plus particulièrement, R₃ représente un groupement protecteur de la fonction hydroxy choisi parmi les radicaux méthoxyméthyle, éthoxy-1 éthyle, benzyloxyméthyle, β-triméthylsilyléthoxyméthyle, tétrahydropyranyle, trichloro-2,2,2 éthoxyméthyle, trichloro-2,2,2 éthoxycarbonyle ou -CH₂-Ph dans lequel Ph représente un radical phényle éventuellement substitué par un ou plusieurs atomes ou radicaux, identiques ou différents, choisis parmi les atomes d'halogène et les radicaux alcoyles contenant 1 à 4 atomes de carbone ou alcoyloxy contenant 1 à 4 atomes de carbone.

Plus particulièrement, lorsque R₂ et R₃ forment ensemble un hétérocycle saturé à 5 ou 6 chaînons, celui-ci représente un cycle oxazolidine substitué en position -2 par 1 ou 2 substituants, identiques ou différents, choisis parmi les atomes d'hydrogène et les radicaux alcoyles contenant 1 à 4 atomes de carbone, alcoxy contenant 1 à 4 atomes de carbone, aralcoyles dont la partie alcoyle contient 1 à 4 atomes de carbone ou aryles, les radicaux aryles étant de préférence des radicaux phényles éventuellement substitués par un ou plusieurs radicaux alcoyloxy contenant 1 à 4 atomes de carbone, et les 2 substituants en position -2 pouvant former avec l'atome de carbone auquel ils sont liés un cycle ayant de 4 à 7 chaînons, ou bien un cycle oxazolidine substitué en position -2 par un radical trihalométhyle ou phényle substitué par un radical trihalométhyle, le symbole R₁ pouvant représenter en outre un atome d'hydrogène.

Plus particulièrement, G₁ représente le radical acétyle ou un groupement protecteur choisi parmi les radicaux trichloro-2,2,2 éthoxycarbonyle ou (trichlorométhyl-2 propoxy)-2 carbonyle.

Plus particulièrement, G₂ représente un groupement protecteur de la fonction hydroxy choisi parmi les radicaux trichloro-2,2,2 éthoxycarbonyle ou (trichlorométhyl-2 propoxy)-2 carbonyle ou trialkylsilyle, dialkylarylsilyle, alkyldiarylsilyle ou triarylsilyle dans lequel chaque partie alkyle contient 1 à 4 atomes de carbone et chaque partie aryle représente de préférence un radical phényle.

Plus particulièrement, X représente un radical acyloxy contenant 1 à 5 atomes de carbone en chaîne droite ou ramifiée ou un radical aroyloxy dans lequel la partie aryle représente un radical phényle éventuellement substitué par 1 à 5 substituants, identiques ou différents, choisis parmi les atomes d'halogène et les radicaux nitro, méthyle ou méthoxy ou bien X représente un atome d'halogène choisi parmi le chlore ou le brome.

Plus particulièrement encore, X représente un radical t.butylcarbonyloxy ou trichloro-2,4,6 benzoyloxy ou un atome de chlore.

Il est connu de préparer des esters de formule générale (I) en opérant dans les conditions décrites par exemple dans les brevets européens EP 0 336 840 et EP 0 336 841 ou dans la demande internationale WO 92/09589. Selon les procédés connus, l'estérification de la baccatine III ou de la désacétyl-10 baccatine III protégée au moyen d'un acide de formule générale : 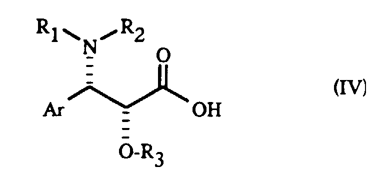 dans laquelle Ar, R₁, R₂ et R₃ sont définis comme précédemment, s'effectue en présence d'un imide tel que le dicyclohexylcarbodiimide et d'une dialkylaminopyridine à une température comprise entre 60 et 90°C.

La mise en oeuvre de ces procédés nécessite l'emploi d'un excès important de l'acide de formule générale (IV) par rapport au dérivé de la baccatine.

De plus, l'emploi d'un agent de condensation tel que le dicyclohexylcarbodiimide peut industriellement poser un certain nombre de problèmes qu'il est important de pouvoir éliminer ou atténuer. En effet, le dicyclohexylcarbodiimide est un réactif coûteux qui, du fait de ses propriétés allergisantes, nécessite des conditions particulières de mise en oeuvre et qui conduit, au cours de sa mise en oeuvre, à la formation de dicyclohexylurée dont l'élimination totale est souvent difficile.

Il a maintenant été trouvé, et c'est ce qui fait l'objet de la présente invention, que les esters de formule générale (I) peuvent être obtenus par estérification de la baccatine III ou de la désacétyl-10 baccatine III convenablement protégée au moyen d'un dérivé activé de formule générale (III) dans des conditions qui permettent de palier aux inconvénients mentionnés ci-dessus.

Selon l'invention, le dérivé activé de formule générale (III), éventuellement préparé in situ, est condensé sur la baccatine III ou la désacétyl-10 baccatine III en présence d'une base, de préférence une base organique azotée, en opérant dans un solvant organique inerte à une température comprise entre 0 et 90°C.

Comme bases organiques azotées qui conviennent particulièrement bien peuvent être citées les amines aliphatiques tertiaires telles que la triéthylamine, la pyridine ou les aminopyridines telles que la diméthylamino-4 pyridine ou la pyrrolidino-4 pyridine.

Comme solvants organiques inertes peuvent être cités les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène ou les xylènes, l'éthylbenzène, l'isopropylbenzène ou le chlorobenzène. D'un intérêt tout particulier sont les hydrocarbures aromatiques.

Généralement, le dérivé activé de formule générale (III) est utilisé en quantité stoechiométrique par rapport au produit de formule (II) mais il peut être avantageux d'utiliser jusqu'à 3 équivalents du produit de formule (III) par rapport au produit de formule (II).

Généralement on utilise au moins 1 équivalent de base organique azotée par rapport au produit de formule générale (II) mis en oeuvre ou par rapport au dérivé de formule générale (III).

De préférence, l'estérification est réalisée à une température voisine de 20°C.

Les dérivés activés de formule générale (III) peuvent être préparés, éventuellement in situ, par action d'un halogénure d'acide de formule générale :

R₅-CO-Y (V)

dans laquelle Y représente un atome d'halogène, de préférence un atome de chlore et R₅ représente un radical alcoyle contenant 1 à 5 atomes de carbone en chaîne droite ou ramifiée ou un radical aryle représentant de préférence un radical phényle éventuellement substitué par 1 à 5 substituants, identiques ou différents, choisis parmi les atomes d'halogène et les radicaux nitro, méthyle ou méthoxy, ou d'un halogénure de thionyle, de préférence le chlorure, sur un acide de formule générale (IV).

Généralement, la réaction s'effectue dans un solvant organique inerte en présence d'une base organique azotée à une température comprise entre 0 et 30°C.

Comme solvants organiques peuvent être utilisés les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène.

Comme bases organiques azotées peuvent être citées les amines aliphatiques tertiaires telles que la triéthylamine, la pyridine ou les aminopyridines comme la diméthylamino-4 pyridine ou la pyrrolidino-4 pyridine.

Généralement on utilise au moins un équivalent de produit de formule générale (V) ou l'halogénure de thionyle par rapport à l'acide de formule générale (IV).

Les esters de formule générale (I) sont particulièrement utiles pour préparer les dérivés du taxane de formule générale : 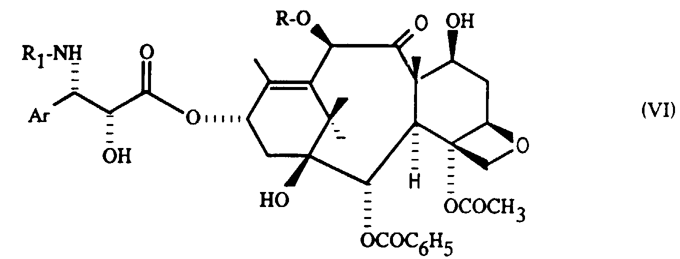 dans laquelle Ar et R₁ sont définis comme précédemment qui présentent des propriétés antileucémiques et antitumorales remarquables.

D'un intérêt tout particulier sont les produits de formule générale (VI) dans laquelle Ar étant défini comme précédemment R représente un atome d'hydrogène ou le radical acétyle et R₁ représente un radical benzoyle ou t.butoxycarbonyle.

Le produit de formule générale (VI) dans laquelle R représente le radical acétyle, R₁ représente un radical benzoyle et Ar représente le radical phényle est connu sous le nom de taxol.

Le produit de formule générale (VI) dans laquelle R représente un atome d'hydrogène, R₁ représente le radical t.butoxycarbonyle et Ar représente le radical phényle, qui est connu sous le nom de Taxotère, fait l'objet du brevet européen EP 0 253 738.

Les produits analogues du Taxotère font l'objet de la demande internationale WO 92/09589.

Selon les significations de R₁, R₂ et R₃, les produits de formule générale (VI) peuvent être obtenus à partir d'un produit de formule générale (I)
- soit directement, lorsque R₁ étant défini comme précédemment, R₂ représente un atome d'hydrogène et R₃ représente un groupement protecteur de la fonction hydroxy, par remplacement des groupements protecteurs R₃, G₁ et G₂ par des atomes d'hydrogène
- soit, lorsque R₁ étant défini comme précédemment, R₂ et R₃ forment ensemble un hétérocycle à 5 ou 6 chaînons, en passant éventuellement intermédiairement par un produit de formule générale : 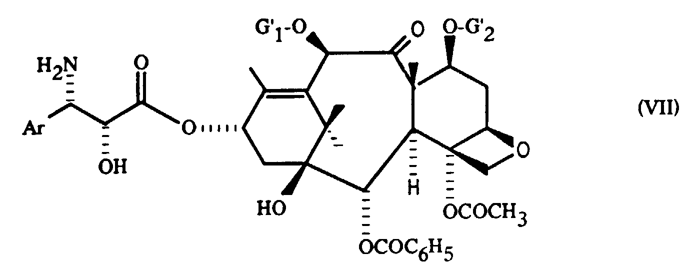 dans laquelle G'₁ et G'₂ sont identiques à G₁ et G₂ et peuvent en outre représenter un atome d'hydrogène qui est soumis à l'action d'un halogénure d'aroyle ou d'un dérivé réactif de formule générale :

   R₄-O-CO-Z (VIII)

   dans laquelle R₄ est défini comme précédemment et Z représente un atome d'halogène ou un reste -O-R₄ ou -O-CO-OR₄ dans lesquels R₄ est défini comme précédemment pour obtenir un produit de formule générale : 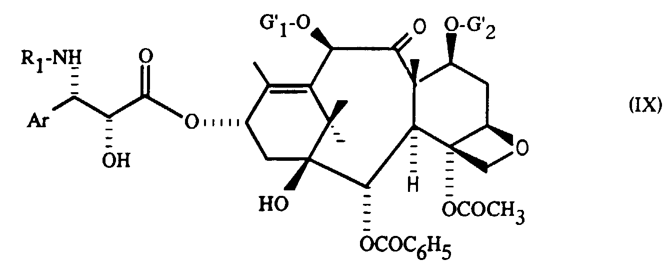 dont les groupements protecteurs G'₁ et G'₂ sont remplacés si nécessaire par des atomes d'hydrogène.

En particulier, lorsque dans la formule générale (I), R₂ et R₃ forment ensemble un cycle oxazolidine gem disubstitué en -2, le produit de formule générale (VI) est obtenu en passant intermédiairement par le produit de formule générale (VII).

Lorsque, dans la formule générale (I), R₁ représente un radical R₄-O-CO- et lorsque R₂ et R₃ forment ensemble un cycle oxazolidine monosubstitué en -2, le produit de formule générale (IX) dans laquelle R₁ = R₄-O-CO- peut être obtenu directement à partir du produit de formule générale (I).

Le produit de formule générale (VII) dans laquelle G'₁ représente un atome d'hydrogène ou un radical acétyle et G'₂ représente un atome d'hydrogène peut être obtenu à partir d'un produit de formule générale (I) dans laquelle R₁ représentant un radical R₄-O-CO- dans lequel R₄ représente un radical alkyle substitué par un ou plusieurs atomes d'halogène, R₂ et R₃ forment ensemble un cycle oxazolidine monosubstitué ou gemdisubstitué en position -2.

Le produit de formule générale (VII) peut aussi être obtenu à partir d'un produit de formule générale (I) dans laquelle R₁ représente un atome d'hydrogène et R₂ et R₃ forment ensemble un cycle oxazolidine substitué en position -2 par un radical trihalométhyle ou phényle substitué par un radical trihalométhyle.

Le remplacement direct des groupements protecteurs R₃, G₁ et G₂ d'un produit de formule générale (I) ou G'₁ et G'₂ d'un produit de formule générale (IX) par des atomes d'hydrogène est effectué par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre, lorsque R₃, G₁ et/ou G₂ représentent un radical trichloro-2,2,2 éthoxycarbonyle, ou par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhyrique aqueux à une température comprise entre 0 et 40°C lorsque R₃, G₁ et/ou G₂ représentent un radical silylé. Lorsque R₃ représente un groupement -CH₂-Ph, il est nécessaire de remplacer ce groupement protecteur par un atome d'hydrogène par hydrogénolyse en présence d'un catalyseur, après avoir remplacé les groupements protecteurs G₁ et G₂ par des atomes d'hydrogène dans les conditions décrites précédemment.

Le produit de formule générale (VII) peut être obtenu à partir d'un produit de formule générale (I) dans laquelle R₂ et R₃ forment ensemble un cycle oxazolidine gem disubstitué en position -2 par traitement au moyen d'acide formique éventuellement dans un alcool tel que l'éthanol ou d'acide chlorhydrique gazeux dans un alcool tel que l'éthanol.

Le produit de formule générale (IX) dans laquelle R₁ représente un radical R₄-O-CO- peut être obtenu directement à partir d'un produit de formule générale (I) dans laquelle R₁ représente un radical R₄-O-CO- et R₂ et R₃ forment ensemble un cycle oxazolidine monosubstitué en position -2 par traitement au moyen d'un acide tel que l'acide méthanesulfonique à une température comprise entre 0 et 40°C.

Le produit de formule générale (VII) dans laquelle G'₁ représente un atome d'hydrogène ou un radical acétyle et G'₂ représente un atome d'hydrogène peuvent être obtenus à partir d'un produit de formule générale (I) dans laquelle R₁ représente un radical R₄-O-CO- dans lequel R₄ représente un radical alcoyle substitué par un ou plusieurs atomes d'halogène, et R₂ et R₃ forment un cycle oxazolidine monosubstitué ou gem disubstitué en position -2 par traitement au moyen de zinc dans l'acide acétique ou par voie électrochimique.

Le produit de formule générale (VII) dans laquelle G'₁ représente un radical acétyle ou un groupement protecteur de la fonction hydroxy et G'₂ représente un groupement protecteur de la fonction hydroxy peut être obtenu à partir d'un produit de formule générale (I) dans laquelle R₁ représente un atome d'hydrogène et R₂ et R₃ forment ensemble un cycle oxazolidine substitué en -2 par un radical trihalométhyle ou phényle substitué par un radical trihalométhyle par traitement au moyen de zinc dans l'acide acétique.

Les exemples suivants illustrent la présente invention.

### EXEMPLE 1

Dans un réacteur de 50 cm3, on introduit, sous atmosphère inerte, à une température voisine de 20°C, 0,321 g de carboxy-5 diméthyl-2,2 phényl-4 (tert.butoxycarbonyl)-3 oxazolidine-1,3-(4S,5R), 0,244 g de chlorure de trichloro-2,4,6 benzoyle, 8 cm3 de toluène anhydre et 0,101 g de triéthylamine. Le mélange réactionnel est laissé pendant 2 heures sous agitation à une température voisine de 20°C. On ajoute alors 0,896 g d'acétoxy-4 benzoyloxy-2α époxy-5β,20 dihydroxy-1β,13α oxo-9 bis-(trichloro-2,2,2 éthoxycarbonyloxy)-7β,10β taxène-11 et 0,122 g de diméthylamino-4 pyridine. Après 20 heures d'agitation à une température voisine de 20°C, le chlorhydrate de triéthylamine formé est séparé par filtration et lavé avec du toluène. La phase toluénique est lavée avec 2 fois 10 cm3 d'eau, séchée sur sulfate de sodium puis concentrée à sec sous pression réduite. Un dosage par chromatographie liquide haute performance montre que le rendement en diméthyl-2,2 phényl-4 (tert.butoxycarbonyl)-3 oxazolidine-1,3-(4S,5R) carboxylate-5 d'acétoxy-4 benzoyloxy-2α époxy-5β,20 hydroxy-1β oxo-9 bis-(trichloro-2,2,2 éthoxycarbonyloxy)-7β,10β taxène-11 yle-13α est de 77 % par rapport à l'alcool transformé et de 63 % par rapport à l'alcool mis en oeuvre.

### EXEMPLE 2

Dans un ballon de 50 cm3 muni d'un système d'agitation magnétique, on introduit, sous atmosphère d'argon, 275 mg d'acide phényl-3 tert.butoxycarbonylamino-3 (éthoxy-1 éthoxy)-2 propionique-(2R,3S) (0,78 mmole) en solution dans 13 cm3 de toluène anhydre. On ajoute ensuite successivement 108,5µl de triéthylamine (0,78 mmole) et 189,5 mg de chlorocarbonyl-1 trichloro-2,4,6 benzène (0,78 mmole). On agite le mélange réactionnel pendant 54 heures à une température voisine de 25°C. Au milieu hétérogène incolore, on ajoute 190,6 mg de diméthylamino-4 pyridine (1,56 mmole). On laisse réagir pendant 5 minutes à une température voisine de 25°C puis on introduit 116 mg (0,13 mmole) d'acétoxy-4 benzoyloxy-2α époxy-5β,20 dihydroxy-1β,13α oxo-9 bis-(trichloro-2,2,2 éthoxycarbonyloxy)-7β,10β taxène-11. On laisse réagir pendant 5 minutes à une température voisine de 25°C puis on chauffe le mélange réactionnel à 72-73°C. On laisse réagir, sous bonne agitation, pendant 64 heures à cette température. Après refroidissement, le mélange réactionnel jaune-orange est dilué par 60 cm3 d'acétate d'éthyle. La phase organique obtenue est lavée 3 fois par 5 cm3 d'une solution aqueuse saturée de bicarbonate de sodium, 5 fois par 5 cm3 d'eau et 2 fois par 5 cm3 d'une solution aqueuse saturée de chlorure de sodium puis est séchée sur sulfate de sodium.

Après filtration et élimination des solvants sous pression réduite (2,7 kPa), on obtient un résidu (488 mg) qui est purifié par chromatographie préparative sur couche mince de silice en éluant avec un mélange éther éthylique-dichlorométhane (5-95 en volumes) et en effectuant 2 passages.

On obtient ainsi 46 mg de dérivé de la baccatine III de départ et 69 mg d'(éthoxy-1 éthoxy)-2 phényl-3 t.butoxycarbonylamino-3 propionate-(2R,3S) d'acétoxy-4 benzoyloxy-2α époxy-5β,20 hydroxy-1β oxo-9 bis-(trichloro-2,2,2 éthoxycarbonyloxy)-7β,10β taxène-11 yle-13α dont la structure est confirmée par le spectre infra-rouge et le spectre de résonance magnétique nucléaire du proton.

Le rendement est de 72 % par rapport à l'alcool mis en oeuvre.

### EXEMPLE 3

En opérant de la même manière que dans l'exemple 1 mais en remplaçant le chlorure de trichloro-2,4,6 benzoyle par 0,120 g de chlorure de pivaloyle on obtient 1,16 g de produit brut dont le dosage par chromatographie liquide haute performance montre que le rendement en diméthyl-2,2 phényl-4 (tert.butoxycarbonyl)-3 oxazolidine-1,3-(4S,5R) carboxylate-5 d'acétoxy-4 benzoyloxy-2α époxy-5β,20 hydroxy-1β oxo-9 bis-(trichloro-2,2,2 éthoxycarbonyloxy)-7β,10β taxène-11 yle-13α est de 98 % par rapport à l'alcool transformé et de 71 % par rapport à l'alcool mis en oeuvre.

### EXEMPLE 4

En opérant de la même manière que dans l'exemple 1 mais en remplaçant le chlorure de trichloro-2,4,6 benzoyle par 0,119 g de chlorure de thionyle et en utilisant 0,202 g de triéthylamine, on obtient 1,36 g de produit brut dont le dosage par chromatographie liquide haute performance montre que le rendement en diméthyl-2,2 phényl-4 (tert.butoxycarbonyl)-3 oxazolidine-1,3-(4S,5R) carboxylate-5 d'acétoxy-4 benzoyloxy-2α époxy-5β,20 hydroxy-1β oxo-9 bis-(trichloro-2,2,2 éthoxycarbonyloxy)-7β,10β taxène-11 yle-13α est de 93 % par rapport à l'alcool transformé et de 31 % par rapport à l'alcool mis en oeuvre.

### EXEMPLE 5

A une solution agitée de 0,353 g d'acide (éthoxy-1 éthoxy)-2 phényl-3 t.butoxycarbonylamino-3 propionique-(2R,3S) et de 0,122 g de diméthylamino-4 pyridine dans 4 cm3 de toluène on ajoute, en 15 minutes et à une température voisine de 20°C, 0,244 g de chlorure de trichloro-2,4,6 benzoyle. Le mélange réactionnel est maintenu pendant 16 heures sous agitation à une température voisine de 20°C. On ajoute 0,448 g d'acétoxy-4 benzoyloxy-2α époxy-5β,20 dihydroxy-1β,13α oxo-9 bis-(trichloro-2,2,2 éthoxycarbonyloxy)-7β,10β taxène-11 et 0,122 g de diméthylamino-4 pyridine. On maintient pendant 20 heures sous agitation. Le dosage par chromatographie liquide haute performance montre que le rendement en (éthoxy-1 éthoxy)-2 phényl-3 t.butoxycarbonylamino-3 propionate-(2R,3S) et (2S,3S) d'acétoxy-4 benzoyloxy-2α époxy-5β,20 hydroxy-1β oxo-9 bis-(trichloro-2,2,2 éthoxycarbonyloxy)-7β,10β taxène-11 est de 58 % par rapport à l'alcool mis en oeuvre et de 100 % par rapport à l'alcool transformé.

Le rapport des deux épimères (2R,3S) / (2S,3S) est de 84/16.

## Revendications

1. Procédé de préparation d'esters de la baccatine III ou de la désacétyl-10 baccatine III de formule générale : 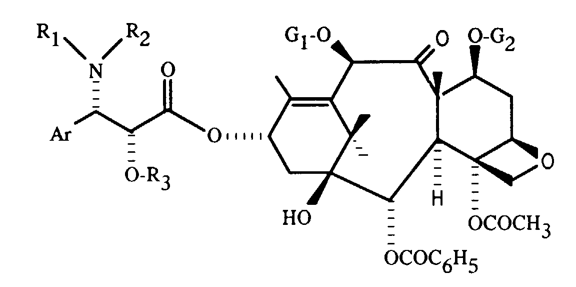 dans laquelle :
- Ar représente un radical aryle éventuellement substitué,
- ou bien a) R₁ représente un radical aroyle éventuellement substitué ou un radical de formule R₄-O-CO-dans laquelle R₄ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 4 à 6 chaînons et éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₂ représente un atome d'hydrogène, et
R₃ représente un groupement protecteur de la fonction hydroxy,
ou bien b) R₁ étant défini comme ci-dessus et pouvant en outre représenter un atome d'hydrogène, R₂ et R₃ forment ensemble un hétérocycle saturé à 5 ou 6 chaînons,
G₁ représente un radical acétyle ou un groupement protecteur de la fonction hydroxy,
G₂ représente un groupement protecteur de la fonction hydroxy,
caractérisé en ce que l'on estérifie la baccatine III ou la désacétyl-10 baccatine III convenablement protégée de formule générale : 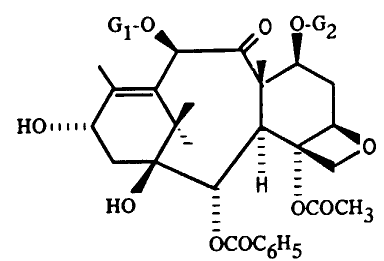 dans laquelle G₁ et G₂ sont définis comme précédemment, au moyen d'un acide activé de formule générale : 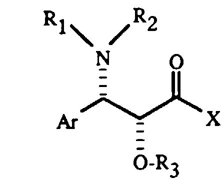 éventuellement préparé in situ, dans laquelle Ar, R₁, R₂ et R₃ sont définis comme précédemment et X représente un radical acyloxy ou aroyloxy ou un atome d'halogène, et isole le produit obtenu.

2. Procédé de préparation selon la revendication 1 caractérisé en ce que R₁, R₂, R₃, G₁ et G₂ étant définis comme dans la revendication 1, Ar et la portion aryle du radical aroyle représenté par R₁, identiques ou différents, représentent un radical phényle ou α- ou β-naphtyle éventuellement substitué, les substituants pouvant être choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, les radicaux alcényles et alcynyles contiennent 3 à 8 atomes de carbone et que les radicaux aryles sont des radicaux phényles ou α- ou β-naphtyles.

3. Procédé de préparation selon la revendication 1 caractérisé en ce que R₁, R₂, R₃, G₁ et G₂ étant définis comme dans la revendication 1, Ar et la portion aryle du radical aroyle représenté par R₁, identiques ou différents, représentent un radical phényle éventuellement substitué par un atome de chlore ou de fluor ou par un radical alcoyle (méthyle), alcoyloxy (méthoxy), dialcoylamino (diméthylamino), acylamino (acétylamino) ou alcoyloxycarbonylamino (t.butoxycarbonylamino).

4. Procédé de préparation selon la revendication 1 caractérisé en ce que R₁, R₂, G₁ et G₂ étant définis comme dans la revendication 1, R₃ représente un groupement protecteur de la fonction hydroxy choisi parmi les radicaux méthoxyméthyle, éthoxy-1 éthyle, benzyloxyméthyle, β-triméthylsilyléthoxyméthyle, tétrahydropyranyle, trichloro-2,2,2 éthoxyméthyle, trichloro-2,2,2 éthoxycarbonyle ou -CH₂-Ph dans lequel Ph représente un radical phényle éventuellement substitué par un ou plusieurs atomes ou radicaux, identiques ou différents, choisis parmi les atomes d'halogène et les radicaux alcoyles contenant 1 à 4 atomes de carbone ou alcoyloxy contenant 1 à 4 atomes de carbone.

5. Procédé selon la revendication 1 caractérisé en ce que Ar, R₁, G₁ et G₂ étant définis comme dans la revendication 1, R₂ et R₃ forment ensemble un cycle oxazolidine substitué en position -2 par 1 ou 2 substituants, identiques ou différents, choisis parmi les atomes d'hydrogène et les radicaux alcoyles contenant 1 à 4 atomes de carbone, alcoxy contenant 1 à 4 atomes de carbone, aralcoyles dont la partie alcoyle contient 1 à 4 atomes de carbone ou aryles, les radicaux aryles étant de préférence des radicaux phényles éventuellement substitués par un ou plusieurs radicaux alcoyloxy contenant 1 à 4 atomes de carbone, et les 2 substituants en position -2 pouvant former avec l'atome de carbone auquel ils sont liés un cycle ayant de 4 à 7 chaînons, ou bien un cycle oxazolidine substitué en position -2 par un radical trihalométhyle ou phényle substitué par un radical trihalométhyle, le symbole R₁ pouvant représenter en outre un atome d'hydrogène.

6. Procédé selon la revendication 1 caractérisé en ce que Ar, R₁, R₂ et R₃ étant définis comme dans la revendication 1, G₁ représente le radical acétyle ou un radical trichloro-2,2,2 éthoxycarbonyle ou (trichlorométhyl-2 propoxy)-2 carbonyle et G₂ représente un radical trichloro-2,2,2 éthoxycarbonyle ou (trichlorométhyl-2 propoxy)-2 carbonyle ou trialkylsilyle, dialkylarylsilyle, alkyldiarylsilyle ou triarylsilyle dans lequel chaque partie alkyle contient 1 à 4 atomes de carbone et chaque partie aryle représente un radical phényle.

7. Procédé selon la revendication 1 caractérisé en ce que X représente un radical acyloxy contenant 1 à 5 atomes de carbone en chaîne droite ou ramifiée ou un radical aroyloxy dans lequel la partie aryle représente un radical phényle éventuellement substitué par 1 à 5 substituants, identiques ou différents, choisis parmi les atomes d'halogène et les radicaux nitro, méthyle ou méthoxy ou bien X représente un atome d'halogène choisi parmi le chlore ou le brome.

8. Procédé selon la revendication 1 caractérisé en ce que X représente un radical t.butylcarbonyloxy ou trichloro-2,4,6 benzoyloxy ou un atome de chlore.

9. Procédé selon la revendication 1 caractérisé en ce que l'on opère en présence d'une base.

10. Procédé selon la revendication 1 caractérisé en ce que l'on opère en présence d'une base organique azotée.

11. Procédé selon la revendication 1 caractérisé en ce que l'on opère en présence d'une base organique azotée choisie parmi les amines aliphatiques tertiaires, la pyridine ou les aminopyridines.

12. Procédé selon la revendication 1 caractérisé en ce que l'on opère dans un solvant organique inerte.

13. Procédé selon la revendication 1 caractérisé en ce que l'on opère dans un solvant organique inerte choisi parmi les éthers, les cétones, les esters, les nitriles, les hydrocarbures aliphatiques éventuellement halogénés et les hydrocarbures aromatiques.

14. Procédé selon la revendication 1 caractérisé en ce que l'on opère à une température comprise entre 0 et 90°C.

15. Procédé selon la revendication 1 caractérisé en ce que l'on opère à une température voisine de 20°C.

16. Procédé selon la revendication 1 caractérisé en ce que l'on opère en présence de 1 à 3 équivalents d'acide activé par rapport à la baccatine III ou à la désacétyl-10 baccatine III convenablement protégée.

17. Procédé selon la revendication 1 caractérisé en ce que l'on opère en présence d'au moins 1 équivalent de base.

18. Les acides activés de formule générale : 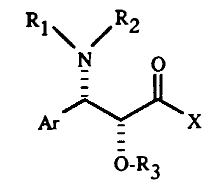 dans laquelle Ar, R₁, R₂, R₃ et X sont définis comme dans la revendication 1.

19. Les acides activés selon la revendication 18 pour lesquels R₁, R₂, R₃ et X étant définis comme dans la revendication 1, Ar est défini comme dans la revendication 2.

20. Les acides activés selon la revendication 18 pour lesquels R₁, R₂, R₃ et X étant définis comme dans la revendication 1, Ar est défini comme dans la revendication 3.

21. Les acides activés selon la revendication 18 pour lesquels Ar étant défini comme dans l'une des revendications 1, 2 ou 3, R₁ et X étant définis comme dans la revendication 1, R₂ et R₃ sont définis comme dans la revendication 5.

22. Les acides activés selon l'une des revendications 18 à 21 pour lesquels X est défini comme dans la revendication 7.

23. Les dérivés activés selon l'une des revendications 18 à 21 pour lesquels X est défini comme dans la revendication 8.

## Patentansprüche

1. Verfahren zur Herstellung von Estern von Baccatin III oder 10-Desacetyl-baccatin III der allgemeinen Formel 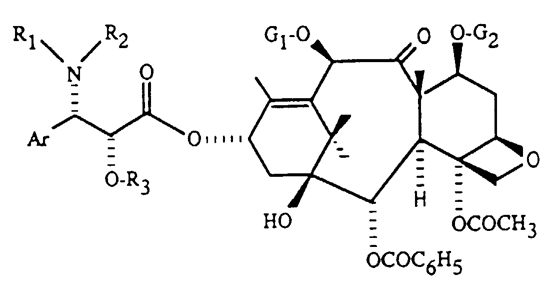 worin
- Ar einen gegebenenfalls substituierten Arylrest bedeutet,
- entweder a) R₁ einen gegebenenfalls substituierten Aroylrest oder einen Rest der Formel R₄-O-CO- bedeutet, worin R₄ bedeutet:
- einen geradkettigen oder verzweigten Alkylrest mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen oder Bicycloalkyl mit 7 bis 10 Kohlenstoffatomen, wobei diese Reste gegebenenfalls substituiert sind durch einen oder mehrere Substituenten, ausgewählt unter den Halogenatomen und den Hydroxyresten, Alkoxy mit 1 bis 4 Kohlenstoffatomen, Dialkylamino, dessen Alkylteil 1 bis 4 Kohlenstoffatome aufweist, Piperidino, Morpholino, Piperazin-1-yl (gegebenenfalls substituiert in 4-Stellung durch einen Alkylrest mit 1 bis 4 Kohlenstoffatomen oder durch einen Phenylalkylrest, dessen Alkylteil 1 bis 4 Kohlenstoffatome aufweist), Cycloalkyl mit 3 bis 6 Kohlenstoffatomen, Cycloalkenyl mit 4 bis 6 Kohlenstoffatomen, Phenyl, Cyano, Carboxy oder Alkoxycarbonyl, dessen Alkylteil bis 4 Kohlenstoffatome enthält,
- oder einen Phenylrest, gegebenenfalls substituiert durch einen oder mehrere Atome oder Reste, ausgewählt unter den Halogenatomen, den Alkylresten mit 1 bis 4 Kohlenstoffatomen oder den Alkoxyresten mit 1 bis 4 Kohlenstoffatomen,
- oder einen gesättigten oder ungesättigten, heterocyclischen stickstoffhaltigen Rest mit 4 bis 6 Gliedern und gegebenenfalls substituiert durch einen oder mehrere Alkylreste mit 1 bis 4 Kohlenstoffatomen,
mit der Maßgabe, daß die Cycloalkyl-, Cycloalkenyl- oder Bicycloalkylreste gegebenenfalls durch einen oder mehrere Alkylreste mit 1 bis 4 Kohlenstoffatomen substituiert sein können,
R₂ ein Wasserstoffatom bedeutet und
R₃ eine Schutzgruppe für die Hydroxyfunktion darstellt,
- oder b) R₁ wie vorstehend definiert ist und außerdem ein Wasserstoffatom bedeuten kann, R₂ und R₃ gemeinsam einen gesättigten Heterocyclus mit 5 oder 6 Gliedern bilden,
G₁ einen Acetylrest oder eine Schutzgruppe für die Hydroxyfunktion bedeutet,
G₂ eine Schutzgruppe für die Hydroxyfunktion bedeutet,
dadurch gekennzeichnet, daß man Baccatin III oder 10-Desacetyl-baccatin III, in geeigneter Weise geschützt, der allgemeinen Formel 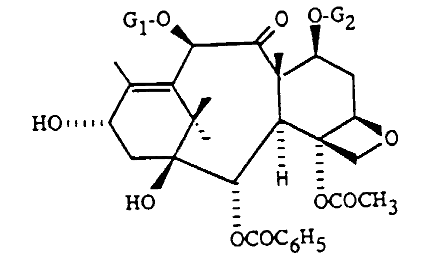 worin G₁ und G₂ wie vorstehend definiert sind, mit einer aktiven Säure der allgemeinen Formel 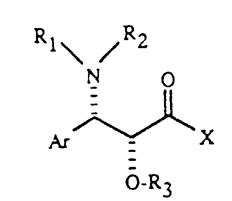 gegebenenfalls hergestellt in situ, worin Ar, R₁, R₂ und R₃ wie vorstehend definiert sind und X einen Acyloxy- oder Aroyloxyrest oder ein Halogenatom bedeutet, verestert und das erhaltene Produkt isoliert.

2. Verfahren gemäß Anspruch 1, dadurch gekennzeichnet, daß R₁, R₂, R₃, G₁ und G₂ wie in Anspruch 1 definiert sind, Ar und der Arylteil des durch R₁ dargestellten Aroylrestes, identisch oder verschieden, einen gegebenenfalls substituierten Phenyl- oder α- oder β-Naphthylrest bedeuten, wobei die Substituenten ausgewählt sein können unter den Halogenatomen (Fluor, Chlor, Brom, Jod) und den Alkyl-, Alkenyl-, Alkinyl-, Aryl-, Arylalkyl-, Alkoxy-, Alkylthio-, Aryloxy-, Arylthio-, Hydroxy-, Hydroxyalkyl-, Mercapto-, Formyl-, Acyl-, Acylamino-, Aroylanino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, mit der Maßgabe, daß die Alkylreste und die Alkylteile der anderen Reste 1 bis 4 Kohlenstoffatome aufweisen, die Alkenyl- und Alkinylreste 3 bis 8 Kohlenstoffatome aufweisen und daß die Arylreste Phenyl- oder α- oder β-Naphthylreste sind.

3. Verfahren gemäß Anspruch 1, dadurch gekennzeichnet, daß R₁, R₂, R₃, G₁ und G₂ wie in Anspruch 1 definiert sind, Ar und der Arylteil des durch R₁ dargestellten Aroylrestes, identisch oder verschieden, einen Phenylrest, gegebenenfalls substituiert durch ein Chlor- oder Fluoratom oder durch einen Alkyl(Methyl)-, Alkoxy(Methoxy)-, Dialkylamino (Dimethylamino)-, Acylamino(Acetylamino)- oder Alkoxycarbonylamino(tert.-Butoxycarbonylamino)-Rest bedeuten.

4. Verfahren gemäß Anspruch 1, dadurch gekennzeichnet, daß R₁, R₂, G₁ und G₂ wie in Anspruch 1 definiert sind, R₃ eine Schutzgruppe für die Hydroxyfunktion darstellt, ausgewählt unter den Methoxymethyl-, 1-Ethoxyethyl-, Benzyloxymethyl-, β-Trimethylsilylethoxymethyl-, Tetrahydropyranyl-, 2,2,2-Trichlorethoxymethyl-, 2,2,2-Trichlorethoxycarbonylresten oder -CH₂-Ph, worin Ph einen Phenylrest, gegebenenfalls substituiert durch einen oder mehrere identische oder verschiedene Atome oder Reste, ausgewählt unter den Halogenatomen und den Alkylresten mit 1 bis 4 Kohlenstoffatomen oder Alkoxy mit 1 bis 4 Kohlenstoffatomen, bedeutet.

5. Verfahren gemäß Anspruch 1, dadurch gekennzeichnet, daß Ar, R₁, G₁ und G₂ wie in Anspruch 1 definiert sind, R₂ und R₃ gemeinsam einen Oxazolidinrest, substituiert in 2-Stellung durch 1 oder 2 identische oder verschiedene Substituenten, ausgewählt unter den Wasserstoffatomen und den Alkylresten mit 1 bis 4 Kohlenstoffatomen, Alkoxyresten mit 1 bis 4 Kohlenstoffatomen, Aralkylresten, deren Alkylteil 1 bis 4 Kohlenstoffatome aufweist, oder Arylresten, wobei die Arylreste bevorzugt Phenylreste sind, die gegebenenfalls durch einen oder mehrere Alkoxyreste mit 1 bis 4 Kohlenstoffatomen substituiert sind, und die 2 Substituenten in 2-Stellung mit dem Kohlenstoffatom, an das sie gebunden sind, einen Ring mit 4 bis 7 Gliedern bilden können, oder einen Oxazolidinring, substituiert in 2-Stellung mit einem Trihalomethylrest oder Phenylrest, der durch einen Trihalomethylrest substituiert ist, bilden, wobei das Symbol R₁ außerdem ein Wasserstoffatom bedeuten kann.

6. Verfahren gemäß Anspruch 1, dadurch gekennzeichnet, daß Ar, R₁, R₂ und R₃ wie in Anspruch 1 definiert sind, G₁ den Acetylrest oder einen 2,2,2-Trichlorethoxycarbonyl- oder 2-(2-Trichlormethylpropoxy)-carbonylrest bedeutet und G₂ einen 2,2,2-Trichlorethoxycarbonyl- oder 2-(2-Trichlormethylpropoxy)-carbonyl- oder Trialkylsilyl-, Dialkylarylsilyl-, Alkyldiarylsilyl- oder Triarylsilylrest, worin jeder Alkylteil 1 bis 4 Kohlenstoffatome aufweist und jeder Arylteil einen Phenylrest bedeutet, darstellt.

7. Verfahren gemäß Anspruch 1, dadurch gekennzeichnet, daß X einen Acyloxyrest mit 1 bis 5 Kohlenstoffatomen in gerader oder verzweigter Kette oder einen Aroyloxyrest, worin der Arylteil einen Phenylrest darstellt, der gegebenenfalls substituiert ist durch 1 bis 5 identische oder verschiedene Substituenten, ausgewählt unter den Halogenatomen und den Nitro-, Methyl- oder Methoxygruppen, bedeutet oder X ein Halogenatom, ausgewählt unter Chlor oder Brom, wiedergibt.

8. Verfahren gemäß Anspruch 1, dadurch gekennzeichnet, daß X einen tert.-Butylcarbonyloxy- oder 2,4,6-Trichlorbenzoyloxyrest oder ein Chloratom bedeutet.

9. Verfahren gemäß Anspruch 1, dadurch gekennzeichnet, daß man in Gegenwart einer Base arbeitet.

10. Verfahren gemäß Anspruch 1, dadurch gekennzeichnet, daß man in Gegenwart einer organischen Stickstoffbase arbeitet.

11. Verfahren gemäß Anspruch 1, dadurch gekennzeichnet, daß man in Gegenwart einer organischen Stickstoffbase, ausgewählt unter tertiären, aliphatischen Aminen, Pyridin oder den Aminopyridinen, arbeitet.

12. Verfahren gemäß Anspruch 1, dadurch gekennzeichnet, daß man in einem inerten, organischen Lösungsmittel arbeitet.

13. Verfahren gemäß Anspruch 1, dadurch gekennzeichnet, daß man in einem inerten, organischen Lösungsmittel, ausgewählt unter den Ethern, den Ketonen, den Estern, den Nitrilen, den gegebenenfalls halogenierten, aliphatischen Kohlenwasserstoffen und den aromatischen Kohlenwasserstoffen arbeitet.

14. Verfahren gemäß Anspruch 1, dadurch gekennzeichnet, daß man bei einer Temperatur zwischen 0 und 90°C arbeitet.

15. Verfahren gemäß Anspruch 1, dadurch gekennzeichnet, daß man bei einer Temperatur von etwa 20°C arbeitet.

16. Verfahren gemäß Anspruch 1, dadurch gekennzeichnet, daß man in Anwesenheit von bis 3 Äquivalenten aktivierter Säure in bezug auf Baccatin III oder auf 10-Desacetylbaccatin III, welches geeignet geschützt ist, arbeitet.

17. Verfahren gemäß Anspruch 1, dadurch gekennzeichnet, daß man in Anwesenheit von zumindest 1 Äquivalent Base arbeitet.

18. Die aktivierten Säuren der allgemeinen Formel 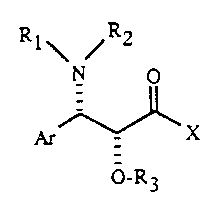 worin Ar, R₁, R₂, R₃ und X wie in Anspruch 1 definiert sind.

19. Die aktivierten Säuren gemäß Anspruch 18, worin R₁, R₂, R₃ und X wie in Anspruch 1 definiert sind, Ar wie in Anspruch 2 definiert ist.

20. Die aktivierten Säuren gemäß Anspruch 18, worin R₁, R₂, R₃ und X wie in Anspruch 1 definiert sind, Ar wie in Anspruch 3 definiert ist.

21. Die aktivierten Säuren gemäß Anspruch 18, worin Ar wie in Anspruch 1, 2 oder 3 definiert ist, R₁ und X wie in Anspruch 1 definiert sind, R₂ und R₃ wie in Anspruch 5 definiert sind.

22. Die aktivierten Säuren gemäß den Ansprüchen 18 bis 21, worin X wie in Anspruch 7 definiert ist.

23. Die aktivierten Säuren gemäß den Ansprüchen 18 bis 21, worin X wie in Anspruch 8 definiert ist.

## Claims

1. Process for the preparation of esters of baccatin III or 10-deacetylbaccatin III of general formula: 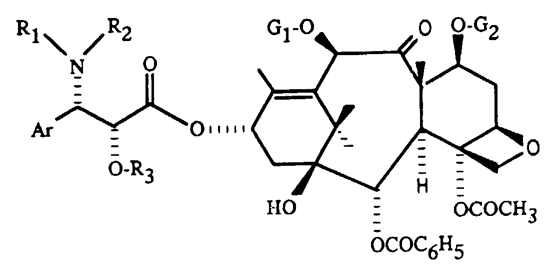 in which:
- Ar represents an optionally substituted aryl radical,
- or else a) R₁ represents an optionally substituted aroyl radical or a radical of formula R₄-O-CO- in which R₄ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms or a bicycloalkyl radical containing 7 to 10 carbon atoms, these radicals optionally being substituted by one or a number of substituents chosen from halogen atoms and hydroxyl radicals, alkyloxy radicals containing 1 to 4 carbon atoms, dialkylamino radicals in which each alkyl part contains 1 to 4 carbon atoms, piperidino radicals, morpholino radicals, 1-piperazinyl radicals (optionally substituted in 4- by an alkyl radical containing 1 to 4 carbon atoms or by a phenylalkyl radical whose alkyl part contains 1 to 4 carbon atoms), cycloalkyl radicals containing 3 to 6 carbon atoms, cycloalkenyl radicals containing 4 to 6 carbon atoms, phenyl radicals, cyano radicals, carboxyl radicals or alkyloxycarbonyl radicals in which the alkyl part contains 1 to 4 carbon atoms,
- or a phenyl radical optionally substituted by one or a number of atoms or radicals chosen from halogen atoms and alkyl radicals containing 1 to 4 carbon atoms or alkyloxy radicals containing 1 to 4 carbon atoms,
- or a saturated or unsaturated nitrogenous heterocyclyl radical containing 4 to 6 members and optionally substituted by one or a number of alkyl radicals containing 1 to 4 carbon atoms,
it being understood that the cycloalkyl, cycloalkenyl or bicycloalkyl radicals can optionally be substituted by one or a number of alkyl radicals containing 1 to 4 carbon atoms,
R₂ represents a hydrogen atom, and
R₃ represents a protective group of the hydroxyl functional group,
or else b) R₁ being defined as above and additionally being able to represent a hydrogen atom, R₂ and R₃ together form a 5- or 6-membered saturated heterocycle,
G₁ represents an acetyl radical or a protective group of the hydroxyl functional group,
G₂ represents a protective group of the hydroxyl functional group,
characterized in that the suitably protected baccatin III or 10-deacetylbaccatin III of general formula: 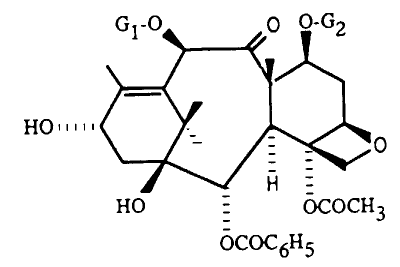 in which G₁ and G₂ are defined as above, is esterified using an activated acid of general formula: 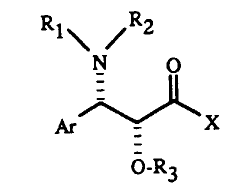 optionally prepared in situ, in which Ar, R₁, R₂ and R₃ are defined as above and X represents an acyloxy or aroyloxy radical or a halogen atom, and the product obtained is isolated.

2. Process of preparation according to claim 1, characterized in that, R₁, R₂, R₃, G₁ and G₂ being defined as in claim 1, Ar and the aryl portion of the aroyl radical represented by R₁, which are identical or different, represent an optionally substituted phenyl or α- or β-naphthyl radical, it being possible for the substituents to be chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and alkyl portions of the other radicals contain 1 to 4 carbon atoms, the alkenyl and alkynyl radicals contain 3 to 8 carbon atoms and that the aryl radicals are phenyl or α- or β-naphthyl radicals.

3. Process of preparation according to claim 1, characterized in that, R₁, R₂, R₃, G₁ and G₂ being defined as in claim 1, Ar and the aryl portion of the aroyl radical represented by R₁, which are identical or different, represent a phenyl radical optionally substituted by a chlorine or fluorine atom or by an alkyl (methyl), alkyloxy (methoxy), dialkylamino (dimethylamino), acylamino (acetylamino) or alkyloxycarbonylamino (t-butoxycarbonylamino) radical.

4. Process of preparation according to claim 1, characterized in that, R₁, R₂, G₁ and G₂ being defined as in claim 1, R₃ represents a protective group of the hydroxyl functional group chosen from methoxymethyl, 1-ethoxyethyl, benzyloxymethyl, β-trimethylsilylethoxymethyl, tetrahydropyranyl, 2,2,2-trichloroethoxymethyl, 2,2,2-trichloroethoxycarbonyl or -CH₂-Ph radicals in which Ph represents a phenyl radical optionally substituted by one or a number of atoms or radicals, which are identical or different, chosen from halogen atoms and alkyl radicals containing 1 to 4 carbon atoms or alkyloxy radicals containing 1 to 4 carbon atoms.

5. Process according to claim 1, characterized in that, Ar, R₁, G₁ and G₂ being defined as in claim 1, R₂ and R₃ together form an oxazolidine ring which is substituted in the 2-position by 1 or 2 substituents, which are identical or different, chosen from hydrogen atoms and alkyl radicals containing 1 to 4 carbon atoms, alkoxy radicals containing 1 to 4 carbon atoms, aralkyl radicals whose alkyl part contains 1 to 4 carbon atoms, or aryl radicals, the aryl radicals preferably being phenyl radicals optionally substituted by one or a number of alkyloxy radicals containing 1 to 4 carbon atoms, and it being possible for the 2 substituents in the 2-position to form, with the carbon atom to which they are bonded, a ring having from 4 to 7 members, or else an oxazolidine ring substituted in the 2-position by a trihalomethyl radical or a phenyl radical substituted by a trihalomethyl radical, it being possible for the symbol R₁ additionally to represent a hydrogen atom.

6. Process according to claim 1, characterized in that, Ar, R₁, R₂ and R₃ being defined as in claim 1, G₁ represents the acetyl radical or a 2,2,2-trichloroethoxycarbonyl or 2-(2-(trichloromethyl)propoxy)carbonyl radical and G₂ represents a 2,2,2-trichloroethoxycarbonyl or 2-(2-(trichloromethyl)propoxy)carbonyl radical or trialkylsilyl, dialkylarylsilyl, alkyldiarylsilyl or triarylsilyl radical in which each alkyl part contains 1 to 4 carbon atoms and each aryl part represents a phenyl radical.

7. Process according to claim 1, characterized in that X represents an acyloxy radical containing 1 to 5 carbon atoms in a straight or branched chain or an aroyloxy radical in which the aryl part represents a phenyl radical optionally substituted by 1 to 5 substituents, which are identical or different, chosen from halogen atoms and nitro, methyl or methoxy radicals or else X represents a halogen atom chosen from chlorine or bromine.

8. Process according to claim 1, characterized in that X represents a t-butylcarbonyloxy or 2,4,6-trichlorobenzoyloxy radical or a chlorine atom.

9. Process according to claim 1, characterized in that the reaction is carried out in the presence of a base.

10. Process according to claim 1, characterized in that the reaction is carried out in the presence of a nitrogenous organic base.

11. Process according to claim 1, characterized in that the reaction is carried out in the presence of a nitrogenous organic base chosen from tertiary aliphatic amines, pyridine or aminopyridines.

12. Process acccording to claim 1, characterized in that the reaction is carried out in an inert organic solvent.

13. Process according to claim 1, characterized in that the reaction is carried out in an inert organic solvent chosen from ethers, ketones, esters, nitriles, optionally halogenated aliphatic hydrocarbons and aromatic hydrocarbons.

14. Process according to claim 1, characterized in that the reaction is carried out at a temperature between 0 and 90°C.

15. Process according to claim 1, characterized in that the reaction is carried out at a temperature in the region of 20°C.

16. Process according to claim 1, characterized in that the reaction is carried out in the presence of 1 to 3 equivalents of activated acid with respect to suitably protected baccatin III or 10-deacetylbaccatin III.

17. Process according to claim 1, characterized in that the reaction is carried out in the presence of at least 1 equivalent of base.

18. The activated acids of general formula: 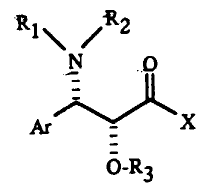 in which Ar, R₁, R₂, R₃ and X are defined as in claim 1.

19. The activated acids according to claim 18 for which, R₁, R₂, R₃ and X being defined as in claim 1, Ar is defined as in claim 2.

20. The activated acids according to claim 18 for which, R₁, R₂, R₃ and X being defined as in claim 1, Ar is defined as in claim 3.

21. The activated acids according to claim 18 for which, Ar being defined as in one of claims 1, 2 or 3, and R₁ and X being defined as in claim 1, R₂ and R₃ are defined as in claim 5.

22. The activated acids according to one of claims 18 to 21 for which X is defined as in claim 7.

23. The activated derivatives according to one of claims 18 to 21 for which X is defined as in claim 8.
